# EUROPÄISCHE PATENTANMELDUNG

(11) **EP 1 870 078 A2**
(43) Veröffentlichungstag der Anmeldung: **26.12.2007**
(21) Anmeldenummer: 07110192.7
(22) Anmeldetag: 13.06.2007
(51) Int. Cl.: A61K 8/19, A61K 8/25, A61K 8/29, A61K 8/67, A61K 8/73, A61K 8/81, A61K 8/97, A61Q 1/02, A61Q 19/08

(54) **Kombinationsset zur dekorativen Pflege der Haut**

(30) Priorität: 19.06.2006 DE 102006028384
(71) Anmelder: Beiersdorf AG, 20253 Hamburg (DE)
(72) Erfinder: Warnke, Dr., Katja, 22547, Hamburg (DE); Kruse, Uta, 20149, Hamburg (DE); Klenner, Katja, 20255, Hamburg (DE); Riedel, Heidi, 22083, Hamburg (DE)

(57) **Zusammenfassung**

Die Erfindung umfasst ein Kombinationsset zur dekorativen Pflege der Haut bestehend aus einem Mehrfachkammerapplikator und mindestens zwei kosmetischen Formulierungen, wobei mindestens eine Zubereitung zur Pflege und mindestens eine Zubereitung zum Schminken der Haut vorgesehen ist.

## Beschreibung

Die Erfindung umfasst ein Kombinationsset zur dekorativen Pflege der Haut umfassend einen Mehrfachkammerapplikator und mindestens zwei kosmetischen Formulierungen, wobei mindestens eine Zubereitung zur Pflege und mindestens eine Zubereitung zum Schminken der Haut vorgesehen ist.

Die Art der Zusammensetzung kosmetischer Zubereitungen findet ihre natürlichen Grenzen, die durch die Verträglichkeit der Einzelkomponenten miteinander sowie der Stabilität von Einzelkomponenten im Trägermedium bestimmt werden. So sind beispielsweise Haut regenerierende Enzyme und Vitamine in kosmetischen Zubereitungen nicht unbegrenzt haltbar. Auch die Kombination unterschiedlich geladener Polymere führt in kosmetischen Formulierungen zu Verklumpung und Ausfällungen.

Es hat nicht an Versuchen gefehlt, derartige Zubereitungen dem Verbraucher zugänglich zu machen. Meist werden dann Teilkomponenten der Formulierungen getrennt verpackt und aufbewahrt. In der Regel werden hierzu sogenannte Zwei-Kammer-Packmittel (engl. dual chamber) verwendet, bei denen die Teilzubereitungen in getrennten Vorratsgefäßen aufbewahrt und dem Packungsbehältnis gleichzeitig über eine gemeinsame Öffnung entnommen werden können. Derartige Zwei-Kammer-Packmittel werden beispielsweise in der WO 2005058272, WO 2003015739, WO 2002022103, WO 2005058259, WO 96/02230, WO 96/37420, WO 98/33477 und der EP 048703 beschrieben. Vielfach werden hierin aber lediglich Reinigungszubereitungen, whitening oder Haarfärbemittel geeignet verpackt.

Herkömmliche Zwei-Kammer-Packmittel haben jedoch eine Reihe von Nachteilen, die ihre Einsatzmöglichkeiten im Kosmetikbereich bisher beschränkten.
Die DE 10342211 beschreibt ein Kosmetikum, welches gebildet wird aus einem Packmittel mit zwei von einander getrennten Vorratskammern, deren Inhalt mit Hilfe zweier gekoppelter Förderpumpen gleichzeitig über eine gemeinsame Austrittsöffnung entnommen werden kann und dadurch gekennzeichnet ist, dass sich das Mengenverhältnis der durch die beiden Pumpen geförderten kosmetischen Zubereitungen zueinander mit Hilfe eines Regelmechanismus frei einstellen lässt, sowie zwei kosmetischen Zubereitungen, die sich jeweils in einer der beiden Vorratskammern des Packmittels befinden und dadurch gekennzeichnet sind, dass sie sich in ihrer Viskosität und/oder Konzentration an kosmetischen und/oder dermatologischen Wirkstoffen von einander unterscheiden.

Die US 6531116 und EP 828670 beschreiben ein Anwendungsverfahren und ein kosmetisches Behandlungsprodukt mit folgenden Merkmalen:
- Verfahren und Produkt zur Behandlung der Haut, umfassend
- eine erste Zusammensetzung, enthaltend mindestens einen ersten Wirkstoff
- eine zweite Zusammensetzung, enthaltend mindestens einen zweiten Wirkstoff
- einen ersten Behälter zum Aufbewahren der ersten Zusammensetzung,
- einen zweiten Behälter zum Aufbewahren der zweiten Zusammensetzung, wobei der erste und der zweite Behälter zusammengefügt sind,
- an den Behältern oder der zugehörigen Verpackung Verbrauchsanweisungen angebracht sind, über die Verwendung
- der ersten und zweiten Zusammensetzung in Kombination miteinander in aufeinander folgender Reihenfolge,
um den ersten und den zweiten Vorteil der Behandlungsvorschrift zu erreichen.

EP 755243 und EP 966264 beschreiben wässrige, flüssige, reinigende und feuchthaltende Zusammensetzungen umfassend zwingend oberflächenaktive Mittel, also Tenside.

EP 916334 beschreibt ebenfalls eine reinigende und Make-up entfernende Zusammensetzung in einem Zweikammerpackmittel

US 5848732 offenbart einen 2-Kompoentendispenser mit Mischvorrichtung, wobei die Mischungsverhältnisse der beiden Komponenten variabel eingestellt werden können.

EP 1541496 beschreibt ein Mehrkammerdispenser zur simultanen Ausbringung zweier getrennt gelagerter Zubereitungen. Vorteil dieses Packmittels ist es, dass es einfach in der Herstellung und im Aufbau konzipiert ist. Wesentlicher Vorteil dieses Dispensers ist eine Verschlussvorrichtung, die eine hermetische Absicherung der separaten Komponenten ermöglicht.

Aufgabe der vorliegenden Erfindung ist es ein einfach zu handhabendes Kosmetikum zur Verfügung zu stellen, das es dem Anwender ermöglicht zeitgleich, mit einer einzigen Applikation zweier unterschiedlicher unvermischter Komponenten eine sowohl hautpflegende als auch dekorative Wirkung zu erzielen.

Wünschenswert ist es insbesondere ein Kosmetikum bereit zu stellen, das keine hautreizenden Substanzen oder Inhaltsstoffe enthält, die die Haut negativ beeinflussen können. Es ist daher wünschenswert ein Kosmetikum ohne Reinigungsmittel bereit zu stellen, das sowohl pflegende und zeitgleich dekorative Wirkungen entfaltet.

Weiterhin wäre es vorteilhaft alternative kosmetische Kits zur dekorativen Pflege der Haut zur Verfügung zu stellen.

Gegenstand der Erfindung ist ein insbesondere kosmetisches Kombinationsset entsprechend Anspruch 1. In den Unteransprüchen sind bevorzugte Ausführungsformen des Sets dargelegt. Erfindungsgemäß ist insbesondere die Verwendung des Sets zur zeitgleichen Pflege und dekorativen kosmetischen Behandlung (Schminken) der Haut.
Eine bevorzugte Verwendung des erfindungsgemäßen Sets ist:
- Einsatz zur Straffung der Haut
- Einsatz zur Festigung der Haut
- Einsatz zur Antifaltenpflege
- Einsatz zu dekorativen Zwecken, wie insbesondere Abdecken von Hautunreinheiten oder Augenringen, Erzielung eines ebenmäßigen Teint und/oder eines natürlichen und strahlenden Teint und jugendliches Aussehens.
Diese Verwendungsmöglichkeiten lassen sich überraschenderweise mit dem erfindungsgemäßen Set gut umsetzen.

Das Kombinationsset umfasst
a.) einen Mehrkammerapplikator zur zeitgleichen und getrennten Ausbringung mindestens zwei im Applikator getrennt enthaltener Zubereitungen, umfassend eine Verschlussvorrichtung, die die Zubereitungen hermetisch voneinander und der Umgebung trennt und die getrennte Ausgabeöffnungen für jede Kammer vorsieht,
b.) wobei mindestens eine der Zubereitungen eine hautpflegende Zubereitung, bevorzugt auf Basis einer O/W-Emulsion, und
c.) mindestens eine andere Zubereitung eine dekorative Zubereitung, bevorzugt auf Basis einer W/S- oder O/W-Emulsion, ist und
d.) die hautpflegenden und die dekorativen Zubereitungen sich in mindestens einem Inhaltstoff voneinander unterscheiden und
e.) die hautpflegenden und die dekorativen Zubereitungen so gewählt werden, dass sie sich bei beim Verteilen, insbesondere auf der Haut, homogen mischen.

Unter homogener Vermischung wird dabei verstanden, dass sich die Zubereitungen zu einer einheitlichen Phase (sowohl farblich als auch von der Textur) vereinigen. Beispielsweise mischen sich Ethanol und Wasser homogen, aber Öl und Wasser nicht.

Die wesentlichen Vorteile des erfindungsgemäßen Sets stellen sich wie folgt dar.
Erfindungsgemäß weisen die Sets einen Mehrkammerapplikator, insbesondere einen Doppelkammerapplikator mit lediglich einem Behältnis auf, welches in mindestens zwei Kammern unterteilt ist, wobei jede Kammer eine Zubereitung - getrennt von der zweiten bzw. weiteren Zubereitungen - aufnimmt. D.h. dass der Applikator aus einem Behältnis mit mindestens zwei getrennten Zubereitungslagern besteht.
Die Ausgabe der Zubereitungen erfolgt bei der Applikation simultan durch Betätigung, meist Drücken, eines Betätigungselementes, welches auf ein Ventil- und/oder Pumpensystem wirkt, wobei für jede Kammer eine Ventil bzw. Ventil-/Pumpenkombination vorhanden ist und die auszugebenden Zubereitungsströme über getrennte Kanäle aus den Kammern nach Aussen geleitet werden.

Der Einsatz der erfindungsgemäßen Mehrkammerapplikatoren sichert eine einfache Handhabung nicht nur bei der Anwendung durch den Konsumenten, sondern zeigt auch bei der Herstellung, insbesondere Befüllung, Vorteile. Insbesondere sind die Materialkosten gegenüber Dispensersystem aus mehrteiligen Behältnissen geringer. Durch die kompaktere Bauweise ist weniger Lagerplatz bzw. Anbietungsfläche notwendig. Die simultane Abfüllung in alle Kammern führt im weiteren zu kürzeren Maschinenzyklen, was einen erhebliche Kostenvorteil gegenüber üblichen Doppelkammerdispensern des Standes der Technik darstellt, die in mehreren Einzelteilen geliefert werden. Die erfindungsgemäßen Mehrkammerapplikatoren lassen sich auf herkömmlichen Produktionslinien befüllen und für den Versand vorbereiten ohne das mehrere Behältnisse zu einem Produkt vereinigt werden müssen.

Erfindungsgemäß besonders geeignet sind
- Mehrkammerschleppkolbendispenser,
- Bag-in-Bottle- bzw. Bag-in-Can-Systeme die mindestens zwei Compartments, insbesondere Folienbeutel, aufweisen und aus denen die Zubereitungen durch Druckbeaufschlagung oder Förderpumpen austragen werden oder
- Mehrkammerpumpdispenser.

Ganz besonders geeignet sind Mehrkammerpumpdispenser, da diese im Gegensatz zu Mehrkammer-Schlepp-Kolben-Systemen in konventioneller topfill-Abfüllung befüllt werden können, die schneller und sicherer funktioniert als die Befüllung von unten.

Bei Mehrkammerdispensern wird das Behältnis mit den separaten Kammern vorzugsweise durch einen aufschnappbares Verschlusselement verschlossen. In das Verschlusselement sind dabei ebenso viele Pumpen - vorzugsweise ein Steigrohr und mindestens ein Ventil aufweisend - wie Behälterkammern integriert, so das zum Verschluß des Mehrkammersdispensers nur ein zuvor konfektionierbares Teil aufgesetzt (geprellt bzw. gepresst) werden muß. Das Verschlusselement kann dabei so gestaltet sein, das flexible Dichtungselemente die Kammeröffnungen gegeneinander abdichten. Solcherlei Mehrkammerdispenser sind besonders bevorzugt.

Durch die hermetische Abdichtung der Kammern untereinander bzw. zur Umgebung, wird eine Mischung der Produkte miteinander verhindert. Die hohe Sicherheit beim Verschließen garantiert so ein funktionstüchtiges Produkt.

Allen erfindungsgemäßen Mehrkammerapplikatoren ist gemein, das auch bei der Austragung der Zubereitungen keine Durchmischung stattfinden kann. Ein Verstopfen der Transportkanäle bzw. der Ausgabeöffnungen durch miteinander reagierende Zubereitungsanteile verschiedener Zubereitungen wird dadurch ausgeschlossen.

Die Durchmischung der separat ausgebrachten Zubereitungen erfolgt erst bei der Anwendung, insbesondere erst bei der Verreibung auf der Haut.

Der erfindungsgemäß bevorzugt verwendete Behälter hat des weiteren eine deutliche Materialersparnis gegenüber anderen Systemen, sowie kostenmäßige und ökologische Vorteile.

Bevorzugt umfasst die hautpflegende Zubereitung, nachfolgend als Zubereitung A gekennzeichnet, eine O/W-Emulsion.
Als Bestandteil oder -teile sind darin hautpflegende Stoffe enthalten. Bevorzugt sind in der hautpflegenden Zubereitung jeweils mindestens ein oder mehrere Bestandteile gewählt aus der Gruppe der hautpflegenden Wirkstoffe, Polymere mit filmbildenden Eigenschaften und Emulgatoren sowie gegebenenfalls Sensorikadditive umfasst.

Bevorzugt sind in der Zubereitung A enthalten:
a.) hautpflegende Wirkstoffe, bevorzugt Ascorbinsäure und deren Derivate, Hyaluronsäure, Kollagen und Wirkstoffe, die die Kollagensynthese stimulieren wie Ginkgo und/oder Mischungen daraus. Bevorzugte Kombinationen sind Ascorbinsäure, Hyaluronsäure und Gingko. Bevorzugt in Einsatzanteilen von 0,01 - 2,5 Gew.%, vorteilhaft auch von 0,01 - 10 Gew.%, bezogen auf die Gesamtmasse der Zubereitung A. Die bevorzugte Kombination aus Ascorbinsäure und deren Derivaten, z.B. Vitamin C Phosphat, Hyaluronsäure und Gingko (Gingko Biloba) spricht verschiedene Mechanismen der Hautstraffung, wie Faltenfüllung, antioxidativer Schutz und Stimulation des hauteigenen Kollagens, an und stellt somit eine bevorzugte Verwendung des erfindungsgegemäßen Sets dar.
b.) Polymere mit filmbildenden Eigenschaften, insbesondere VP/VA Copolymere, Natriumpolystyrensulfonat (Sodium Polystyrene Sulfonate). Die Polymere werden vorteilhaft einzeln oder auch in Kombination in bevorzugten Anteilen von 0,1 - 10 Gew.%, besonders vorteilhaft 0,1 - 2,5 Gew.%, bezogen auf die Gesamtmasse der Zubereitung A eingesetzt. Die bevorzugten Polymere liefern durch die Filmbildung auf der Haut einen sofort spürbaren Straffungseffekt und erhöhen die Transfer-Resistance und den langanhaltenden Effekt der dekorativen Formulierung.
   Auch dadurch ist die bevorzugte Verwendung des Sets zur Hautstraffung gegeben und es zeigt sich die erstaunlicherweise vorteilhafte Lösung der gestellten Aufgaben.
c.) Emulgatoren, wie insbesondere Sucrose Ester, bevorzugt Sucrosepalmitate in Abmischung mit Glycerylstearate und Glycerylstearatcitrate (Handelsname Arlatone V-175). Bevorzugte Anteile der Emulgatoren sind 0,01 - 3 Gew.%, besonders vorteilhaft 0,01 - 1,5 Gew.%, bezogen auf die Gesamtmasse der Zubereitung A.
d.) Zusätzlich können in der Zubereitung A Lichtschutzfilter, bevorzugt Titandioxide (unbeschichtet) und/oder Ethylhexyl Methoxycinnamate, und/oder Sensorikadditive, Effekt- bzw. Farbstoffe, wie Lauroyllysine, Polymethylsilesquioxane, Polymethylmethacrylate Crosspolymer, Nylon, Talkum, Mica oder Stärkederivate. Besonders bevorzugt als Sensorikadditive sind Lauroyl Lysine und/oder Polymethylsilesquioxane der hautpflegenden Zubereitung zu zusetzen.

Weitere bevorzugte Bestandteile der hautpflegenden Zubereitungen werden nachfolgend ausführlicher beschrieben.

Die weitere Zubereitung B, umfasst mindestens einen dekorativen Bestandteil. Die Zubereitung B ist daher auch als eine Foundation oder Make-up Formulierung zu bezeichnen und ist bevorzugt gekennzeichnet durch mindestens einen Farbstoff- bzw. Pigmentanteil, bevorzugt zu einem Anteil von 2 Gew.% und mehr, besonders bevorzugt 10 Gew.% und mehr, bezogen auf die Gesamtmasse der Zubereitung B.
Die Zubereitung B ist gefärbt, z.B. durch Farbstoff bzw. Pigmente. Die Zubereitung B basiert bevorzugt auf einer W/S- oder O/W-Emulsion, die sich durch mindestens einen Inhaltsstoff von der Zubereitung A unterscheidet.

Bevorzugt sind in der Zubereitung B jeweils ein oder mehrere Bestandteile gewählt aus der Gruppe der Farbstoffe oder Pigmente, Emulgatoren sowie gegebenenfalls Füllstoffe, Sensorikadditive und/oder Filmbildner enthalten.
Bevorzugt werden die Bestandteile gewählt aus den Gruppen der
a.) Farbstoffe und/oder Pigmente, bevorzugt sind pigmentäre Farbstoffe auf Basis Eisenoxide, Titandioxide, gecoatet oder nicht gecoatet. Zusätzlich können Effektpigmente wie beispielsweise Perlglanzpigmente oder Metalleffektpigmente enthalten sein.
b.) Emulgatoren wie, z.B. Abil EM 90 (Cetyl PEG/PPG-10/1 Dimethicone) bei W/S-Emulsion, bevorzugt in Anteilen von 0,5 - 10 Gew.%, bevorzugt 2 - 6 Gew.%, bezogen auf die Gesamtmasse der Zubereitung B. Weiter bevorzugt sind Sucrose Ester, bevorzugt Sucrose Palmitate in Abmischung mit Glyceryl Stearate, Glyceryl Stearate Citrate (Arlatone V-175) bei einer O/W-Emulsion. Enthaltend sind dann vorteilhaft 0,01 - 3 Gew.%, besonders vorteilhaft 0,01 - 1.5 Gew.%, an Emulgatoren, bezogen auf die Gesamtmasse der Zubereitung B.
c.) gegebenenfalls Füllstoffe, wie gecoatetes Silica (Silica LD-P),
d.) Sensorikadditive wie Lauroyllysin, Polymethylsilesquioxane, Polymethylmethacrylate, Nylon, Talkum, Mica und/oder Stärkederivate.
e.) Gegebenenfalls können auch Filmbildner enthalten sein.

Besonders bevorzugt sind als Sensorikadditive Lauroyllysine, Silica LD-P bzw. Polymethylsilesquioxane in Anteilen von 0,1 - 10 Gew.% der Einzelkomponenten, besonders bevorzugt 0,5 - 5 Gew.%, bezogen auf die Gesamtmasse der Zubereitung B enthalten.

Optional können in der Foundation auch Wirkstoffe oder Lichtfilter enthalten sein, wie sie in der hautpflegenden Zubereitung beschrieben sind.

Weitere bevorzugte Bestandteile der dekorativen Zubereitungen werden nachfolgend ausführlich beschrieben.

Die aufgeführten bevorzugt genannten Bestandteile der hautpflegenden als auch der dekorativen Zubereitungen können je nach Anwendungszweck individuell und unabhängig voneinander gewählt werden. Beachtet werden muss jedoch die zwingende Erfordernis, dass sich die beiden Zubereitungen durch mindestens einen Inhaltsstoff unterscheiden und so aufeinander abgemischt werden, dass sie sich homogen vermischen lassen. Letzteres ist gerade durch die als bevorzugt genannten Bestandteile und deren Anteile in besonderem Maße gewährleistet.
Bevorzugt umfasst daher das Kombinationsset einen Dispenser zur zeitgleichen und getrennten Ausbringung mindestens zwei im Dispenser getrennt gelagerter Zubereitungen, umfassend eine Verschlussvorrichtung, die die Zubereitungen hermetisch voneinander und der Umgebung trennt,
b.) wobei eine der Zubereitungen eine hautpflegende Zubereitung auf Basis einer O/W-Emulsion darstellt und als hautpflegende Wirkstoffe Ascorbinsäure, bevorzugt Vitamin C Phosphat, Hyaluronsäure und Gingko Biloba, als Polymere mit filmbildenden Eigenschaften VP/VA Copolymere und Natriumpolystyrensulfonate, als Emulgator Sucrosepalmitat in Abmischung mit oder Glycerystearat und Glycerylstearatcitrate und als Sensorikadditive Lauroyllysine und/oder Polymethylsilesquioxane umfasst und
c.) eine andere Zubereitung eine dekorative Zubereitung auf Basis einer W/S- oder O/W-Emulsion darstellt und als Farbstoff pigmentäre Farbstoffe auf Basis Eisenoxide und/oder Titandioxide, gecoatet oder nicht gecoatet, bevorzugt Titandioxid CI 77891, oder Effektpigmente, Perlglanzpigmente oder Metalleffektpigmente, insbesondere gecoatetes Mica, bevorzugt sind Pigmentabmischung aus Cl 77492, 77491, 77499 und 77007, als Emulgatoren eine Mischung aus Cetyl PEG/PPG-10/1 Dimethicone bei W/S-Emulsionen oder Sucrosepalmitate in Abmischung mit Glycerylstearate und Glycerylstearatcitrate, bei O/W-Emulsionen, gewählt werden, und als Füllstoff gecoatetes Silica zugesetzt werden.

Die beiden Zubereitungen unterscheiden sich dabei in mindestens einem Inhaltstoff und lassen sich bei beim Verteilen, insbesondere auf der Haut, homogen mischen.

Die erfindungsgemäße Kombination aus mindestens zwei getrennt gelagerten Zubereitungen, die sich durch mindestens einen Inhaltsstoff unterscheiden, wobei eine hautpflegende Formulierung und die andere eine Foundation darstellt und beide Zubereitungen so gewählt werden, das sie sich beim Verteilen, bevorzugt auf der Haut, homogen mischen, in einem Dispenser, der die zeitgleiche Ausbringung beider Zubereitungen gewährleistet und darüber hinaus durch eine Verschlussvorrichtung, die die Zubereitungen hermetisch voneinander und der Umgebung trennt, und somit Verunreinigungen und Störungen vermeiden hilft, stellt eine überraschend einfache und geniale Lösung der gestellten Aufgaben dar und bietet die Möglichkeit sich zeitgleich neben dem Schminken (Make-up auftragen) gleichzeitig auch gleich die Haut zu pflegen ohne dass die Zubereitungen sich gegenseitig stören, negativ beeinflussen oder sogar unwirksam machen. Durch die Darreichung einer konzentrierten Pflegeformulierung und gleichzeitig einer dekorativen Formulierung, wird die Pflegeleistung (Hautbefeuchtung, Hautglättung) der dekorativen Formulierung effizient erhöht.
Der Vorteil liegt dabei in der zeitgleichen und damit zeitsparenden Anwendung von Hautpflege und Dekoration (Schminken) in Einem. Durch die Darreichung einer konzentrierten Pflegeformulierung gleichzeitig zur dekorativen Formulierung, wird die Pflegeleistung, wie beispielsweise Hautbefeuchtung oder Hautglättung, der dekorativen Formulierung effizient erhöht. Es wird ein gleichzeitiger Effekt von innen, wie Pflege und Straffung durch kosmetische Wirkstoffe, mit einem sofort sichtbaren Effekt von außen, wie ebenmäßige, gepflegte Haut durch Make-up, kombiniert.
Der erfindungsgemäße Set wird verwendet um die Effekte der Hautstraffung und/oder Festigung der Haut, Antifaltenwirkung, Abdecken von Hautunreinheiten oder Augenringen, ebenmäßiger Teint, natürlicher und strahlender Teint, jugendliches Aussehen, lang anhaltend, Kaschierung kleiner Fältchen, zu erreichen. Dass dies erfindungsgemäß gelingt und darüber hinaus auch einfacher, zeitsparender und effektiver, wird insbesondere durch die nachfolgenden Beispiele eindrucksvoll bestätigt.

Die erfindungsgemäße Kombination aus Dispenser und den mindesten zwei kosmetischen Zubereitungen, wobei eine mindestens eine hautpflegende und die andere Zubereitung eine dekorative Wirkung auf der Haut entfalten, ermöglicht erst die in der Aufgabenstellung formulierte einfache, anwendungsfreundliche und zeitgleiche Pflege und Dekoration der Haut.

Um keinerlei Hautschädigungen hervorzurufen umfasst das erfindungsgemäße Kombinationsset keine Reinigungsmittel, keine Tenside und ist nicht zum Reinigen der Haut geeignet.

Ebenso ist es wichtig, dass beide kombinierten Zubereitungen nicht in aufeinander folgender Reihenfolge zur Anwendung kommen, sondern zeitgleich aufgebracht werden.
Ebenso wesentlich ist, das sich die beiden Zubereitungen, die hautpflegende Zubereitung und die Foundation sich beim Verteilen homogen mischen. Die Verteilung erfolgt vorteilhafterweise direkt auf der Haut. Dies gelingt erstaunlicherweise besonders vorteilhaft aufgrund der als bevorzugt benannten Bestandteile.

Zur Anwendung werden die erfindungsgemäßen Zubereitungen in der für Kosmetika üblichen Weise auf die Haut in ausreichender Menge aufgebracht. Die erfindungsgemäßen kosmetischen Zubereitungen können wie üblich zusammengesetzt sein und zur Behandlung, d.h. zur Pflege der Haut und als Schminkprodukt in der dekorativen Kosmetik dienen.

Die kosmetischen Zubereitungen gemäß der Erfindung stellen bevorzugt Emulsionen dar.

Es ist besonders bevorzugt im Sinne der vorliegenden Erfindung, wenn die eine der beiden Kammern eine Zubereitung zur Pflege der Haut (Zubereitung A, im folgenden auch als Pflegeemulsion bezeichnet) und die andere der beiden Kammern eine Zubereitung zum Schminken der Haut (Zubereitung B, im folgenden auch als Foundation bezeichnet) enthält.

Es ist erfindungsgemäß besonders vorteilhaft, wenn sowohl die Pflegeemulsion als auch die Foundation in Form einer Öl-in-Wasser-Emulsion (O/W-Emulsion) vorliegen. In einer anderen erfindungsgemäß vorteilhaften Ausführungsform stellt die Pflegeemulsion eine O/W-Emulsion dar und die Foundation ist eine Wasser-in-Silicon- (W/S-) Emulsion.

Für den Fall, dass eine oder beide der Zubereitungen in Form einer O/W-Emulsion vorliegen, werden der oder die O/W-Emulgatoren erfindungsgemäß vorzugsweise aus der folgenden Gruppe gewählt, wobei die zuvor genannten Emulgatoren bevorzugt sind:
Sucroseester - wie insbesondere Sucrosepalmitat - in Kombination mit Glycerylstearat und Glycerylstearatcitrat (beispielsweise erhältlich unter dem Handelsnamen Arlatone V-175 von der Fa. Uniqema), aber auch Glycerylstearat in Kombination mit Ceteareth-20 und/oder Ceteareth-25, Ceteareth-6 in Kombination mit Stearylalkohol, Cetylstearylalkohol in Kombination mit PEG-40-Ricinusöl und Natriumcetylstearylsulfat, Triceteareth-4 Phosphat, Glycerylstearat, Natriumcetylstearylsulfat, Lecithin Trilaureth-4 Phosphat, Laureth-4 Phosphat, Stearinsäure, Propylenglycolstearat SE, PEG-25-hydriertes Ricinusöl, PEG-54-hydriertes Ricinusöl und/oder PEG-6 Caprylsäure/Caprinsäureglyceride, Glyceryloleat in Kombination mit Propylenglycol, PEG-9-Stearat, PEG-20 Stearat, PEG-30-Stearat, PEG-40-Stearat, PEG-100-Stearat, Ceteth-2, Ceteth-20, Polysorbate-20, Polysorbate-60, Polysorbate-65 und/oder Polysorbate-100, Glycerylstearat in Kombination mit PEG-100 Stearat, Glycerylmyristat, Glyceryllaurat, PEG-40-Sorbitanperoleat, Laureth-4, Ceteareth-3 und/oder Isostearylglycerylether, Cetylstearylalkohol in Kombination mit Natrium Cetylstearylsulfat, Laureth-23 und/oder Steareth-2, Glycerylstearat in Kombination mit PEG-30 Stearat, PEG-40-Stearat, Glycol Distearat, PEG-22-Dodecyl Glycol Copolymer, Polyglyceryl-2-PEG-4-Stearat, Ceteareth-12, Ceteareth-20, Ceteareth-30, Methyl-glucosesesquistearat, Steareth-10 und/oder PEG-20-Stearat, Steareth-2 in Kombination mit PEG-8 Distearat, Steareth-21, Steareth-20, Isosteareth-20, PEG-45/ Dodecylglycol-Copolymer, Methoxy-PEG-22/Dodecylglycol-Copolymer, PEG-40-Sorbitanperoleat, PEG-40-Sorbitanperisostearat, PEG-20-Glycerylstearat, PEG-20-Glycerylstearat, PEG-8-Bienenwachs, Polyglyceryl-2-laurat, Isostearyldiglycerylsuccinat, Stearamidopropyl-PGdimoniumchloridphosphat, Glycerylstearat SE, Ceteth-20, Triethylcitrat, PEG-20-Methylglucosesesquistearat, Glycerylstearatcitrat, Cetylphosphat, Cetearyl Sulfat, Sorbitansesquioleat, Triceteareth-4-Phosphat, Trilaureth-4-Phosphat, Polyglyceryl-methylglucosedistearat, Kaliumcetylphosphat, Polyglyceryl-3 Methylglucose Distearate Isosteareth-10, Polyglyceryl-2-sesquiisostearat, Ceteth-10, Oleth-20 und/oder Isoceteth-20, Glycerylstearat in Kombination mit Ceteareth-20, Ceteareth-12, Cetylstearylalkohol und/oder Cetylpalmitat, Cetylstearylalkohol in Kombination mit PEG-20 Stearat, PEG-30-Stearat, PEG-40-Stearat und/oder PEG-100-Stearat.

Erfindungsgemäß besonders vorteilhafte O/W-Emulgatoren sind Sucroseester - wie insbesondere Sucrosepalmitat - in Kombination mit Glycerylstearat und Glycerylstearatcitrat (beispielsweise erhältlich unter dem Handelsnamen Arlatone V-175 von der Fa. Uniqema) Glycerylstearat, PEG-40 Stearat, Triglycerinmethylglucosedistearat, Polyglyceryl-3 Methylglucose Distearate, Polyethylenglycol(21)stearylether, Polyethylenglycol(2)stearylether, Cetearylglucosid, Glycerylstearatcitrat, Natriumcetearyl-sulfat, Cetearylalkohol und/oder Sorbitanstearat .
Bevorzugte Anteile der Emulgatoren sind 0,01 - 3 Gew.%, besonders vorteilhaft 0,01 - 1,5 Gew.%, bezogen auf die Gesamtmasse der Zubereitung A.

Die kosmetische Zubereitung B, die Foundations, können vorteilhaft einen oder mehrere Siliconemulgatoren enthalten, insbesondere wenn sie in Form von W/S-Emulsionen vorliegen.

Erfindungsgemäß können der oder die Siliconemulgatoren vorteilhaft aus der Gruppe der grenzflächenaktiven Substanzen aus der Gruppe der Alkylmethiconcopolyole und/oder Alkyl-Dimethiconcopolyole gewählt werden, insbesondere aus der Gruppe der Verbindungen, welche gekennzeichnet sind durch die folgende chemische Struktur: 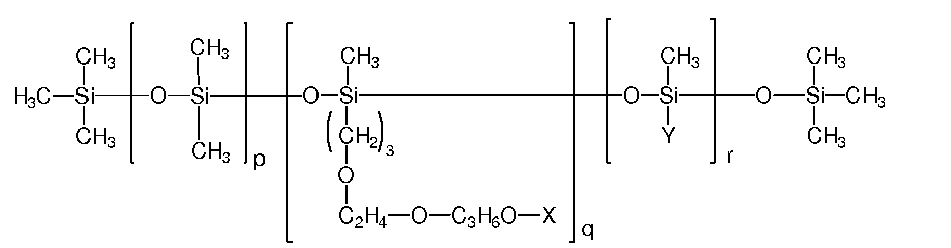 bei welcher X und Y unabhängig voneinander gewählt werden aus der Gruppe H sowie der verzweigten und unverzweigten Alkylgruppen, Acylgruppen und Alkoxygruppen mit 1 - 24 Kohlenstoffatomen, p eine Zahl von 0 - 200 darstellt, q eine Zahl von 1 - 40 darstellt, und r eine Zahl von 1 - 100 darstellt.

Ein Beispiel für besonders vorteilhaft im Sinne der vorliegenden Erfindung zu verwendende Siliconemulgatoren sind Dimethiconcopolyole, welche von der Gesellschaft Degussa AG unter den Warenbezeichnungen ABIL® B 8842, ABIL® B 8843, ABIL® B 8847, ABIL® B 8851, ABIL® B 8852, ABIL® B 8863, ABIL® B 8873 und ABIL® B 88183 verkauft werden.

Ein weiteres Beispiel für besonders vorteilhaft im Sinne der vorliegenden Erfindung zu verwendende Siliconemulgatoren, wie bereits erwähnt, ist das Cetyl PEG/PPG-10/1 Dimethicone, welches von der Gesellschaft Degussa AG unter der Warenbezeichnung ABIL® EM 90 verkauft wird.

Ein weiterer besonders vorteilhaft im Sinne der vorliegenden Erfindung zu verwendender Siliconemulgator ist das Cyclomethicon Dimethiconcopolyol, welches von der Gesellschaft Goldschmidt AG unter der Warenbezeichnung ABIL® EM 97 verkauft wird.

Weiterhin hat sich als ganz besonders vorteilhaft der Emulgator Laurylmethiconcopolyol herausgestellt, welcher unter der Warenbezeichnung Dow Corning® 5200 Formulation Aid von der Gesellschaft Dow Corning Ltd. erhältlich ist.

Die Gesamtmenge an Siliconemulgatoren in der oder den erfindungsgemäßen kosmetischen Zubereitungen wird vorteilhaft aus dem Bereich von 0,1 bis 10,0 Gew.-%, bevorzugt 0,5 bis 5,0 Gew.-% gewählt, besonders bevorzugt 2 - 4 Gew.%, jeweils bezogen auf das Gesamtgewicht der dekorativen Zubereitungen.

Die kosmetischen Zubereitungen B gemäß der Erfindung können ferner vorteilhaft einen oder mehrere grenzflächenaktive Polyether enthalten, insbesondere wenn die Zubereitungen in Form von W/S-Emulsionen vorliegen:

Vorteilhaft im Sinne der vorliegenden Erfindung sind die in den Chemical Abstracts mit der Registraturnummer 78336-31-9 bezeichneten Polyether, welche die chemische Bezeichnung Polyethylenglycoldi(polydodecylenglycol)ether tragen und beispielsweise als PEG-45 (dodecyl glycol) copolymer unter der Marke Elfacos® ST 9 (mittleres Molekulargewicht ca. 4 000 g/mol) bzw. als PEG-22 (dodecyl glycol) copolymer unter der Marke Elfacos® ST 37 (mittleres Molekulargewicht ca. 2 300 g/mol) von der Gesellschaft Akzo Nobel Chemicals GmbH verkauft werden.

Die Gesamtmenge an den grenzflächenaktiven Polyethern in den fertigen kosmetischen Zubereitungen wird vorteilhaft aus dem Bereich von 0,1 bis 10 Gew.-%, bevorzugt 0,25 bis 5,0 Gew.-% insbesondere 0,75 bis 3,5 Gew.-% gewählt, jeweils bezogen auf das Gesamtgewicht der dekorativen Zubereitungen B.

Es ist ferner vorteilhaft im Sinne der vorliegenden Erfindung, Polyethylenglycol-30-Dipolyhydroxystearat (PEG-30-Dipolyhydroxystearat), welches von der Gesellschaft Uniqema unter der Handelsbezeichnung ARLACEL® P135 verkauft wird, als grenzflächenaktive Substanz zu verwenden, insbesondere wenn die Zubereitung in Form einer Wasser-in-Öl-Emulsion vorliegt. Darüber hinaus kann PEG-30-Dipolyhydroxystearat - ebenso wie andere W/O-Emulgatoren - aber auch vorteilhaft als Filmbildner eingesetzt werden, z. B. um die Abriebfestigkeit (Transferresistenz) der Zubereitungen zu verbessern bzw. um Long-lasting Effekte zu erreichen.
Dieser Effekt lässt sich durch die in der Literatur bekannten Standard-Methoden und Home-in-use Daten belegen.

Die erfindungsgemäße Foundation (Zubereitung B) kann, in einer speziellen Ausführungsform vorteilhaft auch in Form einer Wasser-in-Öl-Emulsion (W/O-Emulsion) vorliegen. In diesem Fall sind die folgende Emulgatoren erfindungsgemäß bevorzugt:

| Handelsname | INCI-Name |
|---|---|
| Lameform TGI | Polyglyceryl-3 Diisostearate |
| Isolan Gl 34 | Polyglyceryl-4 Isostearate |
| Dehymuls PGPH | Polyglyceryl-2 Dipolyhydroxystearate |
| Arlacel P 135 | PEG-30 Dipolyhydroxystearate |
| Eucerit PA | Lanolin Alcohol |
| Atlas G-1 049 | PEG-40 Sorbitan Perisostearate |
| Abil EM 90 | Cetyl Dimethicone Copolyol |
| Arlacel 989 | PEG-7 Hydrogenated Castor Oil |
| Elfacos ST 9 | PEG 45/Dodeceyl Glycol Copolymer |
| Elfacos ST 37 | PEG 22/Dodeceyl Glycol Copolymer |
| Isostearinsäure PP | Pentaerythrityl Isostearate |
| Imwitor 780 K | Isostearyl Diglyceryl Succinate |
| Arlacel 987 | Sorbitan Isostearate |
| Hostacerin DGl | Polyglyceryl-2 Sesquiisostearate |
| Tegin ISO | Glyceryl Isostearate |
| Arlacel 60 | Sorbitan Stearate |
| Tegin M | Glyceryl Stearate |
| Arlantone G | PEG-25 Hydrogenated Castor Oil |
| Arlantone T | PEG-40 Sorbitan Peroleate |
| Arlacel 80 | Sorbitan Oleate |
| | Cera Microcristallina + Paraffinum Liquidum + Ozokerite+ Hydrogenated Castor Oil + Glyceryl Isostearate + Polyglyceryl-3 Oleate |
| Atlas G-1 049 | PEG-40 Sorbitan Perisostearate |
| ABIL WS 08 | Cetyl Dimethicone Copolyol + Hexyl Laurate + Polyglyceryl-3 Oleate + Cetyl Dimethicone |
| Hostacerin DGO | Polyglyceryl-2 Sesquioleate |
| Abil WE 09 | Cetyl Dimethicone Copolyol (+) Polyglyceryl-4 Isostearate (+) Hexyl Laurate |
| Abil EM 90 | Cetyl PEG/ PPG- 10/1- Dimethicone |
| Abil EM 97 | Dimethicone Copolyol (+) Cyclomethicone |
| Isolan GO 33 | Polyglyceryl-3 Oleate |
| Montanov WO 18 | Isostearyl Alcohol (+) Isostearyl Glucoside |
| Cremophor GO 32 | Polyglyceryl-3 Dioleate |
| Olivem 900 | Sorbitan Oleate |
| Sisterna SP01-C | Sucrose Distearate |
| Sisterna SP10-C | Sucrose Distearate |
| Dehymuls B | Polyglyceryl-3 Diisostearate (+) Glyceryl Oleate |
| Emulsogen SRH | Rapeseed Sorbitols |
| Emulsogen SRO | Rapeseed Sorbitols |
| Rylo PG 11 | Polyglyceryl Dimer Soyate |
| Grindstedt PS 401 (Dermofeel PR) | Polyglyceryl Polyricinoleate |
| Isolan PDI | Polyglyceryl-3 Dimerate |
| Arlacel 986 | Glyceryl Sorbitan Stearate |
| Decaglyn 5-HS | Polyglyceryl-10 Hydroxystearate |

Ganz besonders bevorzugt ist es, wenn der oder die W/O-Emulgatoren gewählt werden aus der Gruppe PEG-30 Dipolyhydroxystearat, Polyglyceryl-3-Diisostearat, PEG-40 Sorbitan Perisostearate.

Die Ölphasenbestandteile der erfindungsgemäßen Zubereitungen A und/oder B werden vorteilhaft gewählt aus der Gruppe der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Besonders vorteilhaft im Sinne der vorliegenden Erfindung ist Capryl-Caprinsäuretriglycerid (INCI: Caprylic/capric Triglyceride) zu verwenden.

Die Fettsäuretriglyceride können vorteilhaft gewählt werden aus der Gruppe der synthetischen, halbsynthetischen und natürlichen Öle, wie z. B. Olivenöl, Sonnenblumenöl, Sojaöl, Erdnussöl, Rapsöl, Mandelöl, Palmöl, Kokosöl, Rizinusöl, Weizenkeimöl, Traubenkernöl, Distelöl, Nachtkerzenöl und dergleichen mehr.

Die Ölphase kann im Sinne der vorliegenden Erfindung ferner vorteilhaft Substanzen aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen sowie aus der Gruppe der Ester aus aromatischen Carbonsäuren und gesättigten und/oder ungesättigten, verzweigten und/oder unverzweigten Alkoholen einer Kettenlänge von 3 bis 30 C-Atomen enthalten. Solche Esteröle können dann vorteilhaft gewählt werden aus der Gruppe Isopropylpalmitat, Isopropylstearat, n-Butylstearat, n-Hexyllaurat, Isooctylstearat, Isononylstearat, Isononylisononanoat, 2-Ethylhexylpalmitat, 2-Hexyldecylstearat, 2-Octyldodecylpalmitat, Erucylerucat sowie synthetische, halbsynthetische und natürliche Gemische solcher Ester, wie z. B. Jojobaöl.

Es ist ferner vorteilhaft im Sinne der vorliegenden Erfindung, wenn die Zubereitungen ein oder mehrere flüchtige Lipide enthalten, z. B. aus der Gruppe Phenyltrimethicon (INCI-Bezeichnung: Phenyl Trimethicone, CAS 2116-84-9), Cyclomethicone (INCI-Bezeichnung: Cyclomethicone, CAS 556-67-2 u. 69430-24-6) - insbesondere Octamethylcyclotetrasiloxan, Cyclomethicone D5 und/oder Cyclomethicone D6 - und C12-20 Isoparaffin bzw. Isohexadekan.

Erfindungsgemäß besonders vorteilhaft sind Siliconöle sowie Siliconölabmischungen bestehend aus flüchtigen Siliconölen (wie Cyclomethicone) und nicht-flüchtigen Siliconölen (Dimethicone, Phenytrimethicone), insbesondere wenn die Zubereitung in Form einer W/S-Emulsion vorliegt.

Die Bestandteile der Ölphase können beliebige Abmischungen von Öl-, Fett- und/oder Wachskomponenten darstellen.

Es ist vorteilhaft im Sinne der vorliegenden Erfindung, wenn die Zubereitungen einen oder mehrere Glycerinester, insbesondere Glycerinester von α-Hydroxycarbonsäuren und gesättigten Fettsäuren enthalten, wobei die Gesamtmenge der Glycerinester in den fertigen kosmetischen Zubereitungen vorteilhaft aus dem Bereich von 0,1 bis 10,0 Gew.-%, bevorzugt 0,5 bis 6,0 Gew.-% gewählt wird, jeweils bezogen auf das Gesamtgewicht der Zubereitungen.

Die kosmetischen Zubereitungen gemäß der Erfindung können kosmetische Hilfsstoffe enthalten, wie sie üblicherweise in solchen Zubereitungen verwendet werden, z. B. Konservierungsmittel, Konservierungshelfer, Komplexbildner, Bakterizide, Parfüme, Substanzen zum Verhindern oder Steigern des Schäumens, Verdickungsmittel, anfeuchtende und/oder feuchthaltende Substanzen, Füllstoffe, die das Hautgefühl verbessern, oder andere übliche Bestandteile einer kosmetischen Formulierung wie Alkohole, Polyole, Polymere, Schaumstabilisatoren, Elektrolyte, organische Lösungsmittel oder Siliconderivate.

Geeignete Konservierungsmittel im Sinne der vorliegenden Erfindung sind beispielsweise Formaldehydabspalter (wie z. B. DMDM Hydantoin, welches beispielsweise unter der Handelsbezeichnung Glydant^{™} von der Fa. Lonza erhältlich ist), lodopropylbutylcarbamate (z. B. die unter den Handelsbezeichnungen Glycacil-L, Glycacil-S von der Fa. Lonza erhältlichen und/oder Dekaben LMB von Jan Dekker), Parabene (d. h. p-Hydroxybenzoesäurealkylester, wie Methyl-, Ethyl-, Propyl- und/oder Butylparaben), Phenoxyethanol, Ethanol, Benzoesäure und dergleichen mehr. Üblicherweise umfasst das Konservierungssystem erfindungsgemäß ferner vorteilhaft auch Konservierungshelfer, wie beispielsweise Octoxyglycerin, Glycine Soja etc.

Obwohl die genannten Konservierungsmittel an sich in den erfindungsgemäßen Zubereitungen vorteilhaft eingesetzt werden können, sind ganz besonders bevorzugt solche Zubereitungen, welche frei von Parabenen und/oder frei von Formaldehydabspaltern sind.

Vorteilhafte Komplexbildner im Sinne der vorliegenden Erfindung sind beispielsweise EDTA, [S,S]-Ethylendiamindisuccinat (EDDS), welches beispielsweise unter der Handelsbezeichnung Octaquest von der Fa. Octel erhältlich ist, Pentanatrium-Ethylendiamintetramethylenphosphonat, welches z. B. unter dem Handelsnamen Dequest 2046 von der Fa. Monsanto erhältlich ist und/oder Iminodibersteinsäure, welche u. a. von der Fa. Bayer AG unter den Handelsnamen Iminodisuccinat VP OC 370 (ca. 30% ige Lösung) und Baypure CX 100 fest erhältlich ist.

Besonders vorteilhafte Zubereitungen werden ferner erhalten, wenn als Zusatz- oder Wirkstoffe Antioxidantien eingesetzt werden. Erfindungsgemäß enthalten die Zubereitungen vorteilhaft eines oder mehrere Antioxidantien. Als günstige, aber dennoch fakultativ zu verwendende Antioxidantien können alle für kosmetische Anwendungen geeigneten oder gebräuchlichen Antioxidantien verwendet werden.

Besonders vorteilhaft im Sinne der vorliegenden Erfindung können wasserlösliche Antioxidantien eingesetzt werden, wie beispielsweise Vitamine, z. B. Ascorbinsäure (Vitamin C) und deren Derivate. Diese bevorzugt genannten Bestandteile werden ebenso als hautpflegende Wirkstoffe erfindungsgemäß gewählt und sind daher bevorzugt der hautpflegenden Zubereitung zu zu setzen.

Bevorzugte Antioxidantien sind ferner Vitamin E und dessen Derivate sowie Vitamin A und dessen Derivate.

Die Menge der Antioxidantien (eine oder mehrere Verbindungen) in den Zubereitungen beträgt vorzugsweise 0,001 bis 30 Gew.-%, besonders bevorzugt 0,05 bis 20 Gew.-%, insbesondere 0,1 bis 10 Gew.-%, bezogen auf das Gesamtgewicht der Zubereitung.

Sofern Vitamin E oder Vitamin C und/oder dessen Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Sofern Vitamin A bzw. Vitamin-A-Derivate, bzw. Carotine bzw. deren Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Es ist insbesondere vorteilhaft, wenn die kosmetische Zubereitung A gemäß der vorliegenden Erfindung kosmetische hautpflegende Wirkstoffe enthalten, wobei bevorzugte Wirkstoffe Antioxidantien sind, welche die Haut vor oxidativer Beanspruchung schützen können.

Weitere vorteilhafte hautpflegende Wirkstoffe im Sinne der vorliegenden Erfindung sind natürliche Wirkstoffe und/oder deren Derivate, wie z. B. Ginkgo Biloba bzw. Ginkgo-Extrakte, Hyaluronsäure, Collagen, alpha-Liponsäure, Phytoen, D-Biotin, Coenzym Q10, alpha-Glucosylrutin, Carnitin, Carnosin, natürliche und/oder synthetische Isoflavonoide, Kreatin, Taurin und/oder β-Alanin sowie 8-Hexadecen-1,16-dicarbonsäure (Dioic acid, CAS-Nummer 20701-68-2; vorläufige INCI-Bezeichnung Octadecendioic acid).

Erfindungsgemäße Zubereitungen, welche z. B. bekannte Faltenfüllwirkstoffe wie Hyaluronsäure und/oder Derivate und dergleichen enthalten, eignen sich insbesondere vorteilhaft zur kosmetischen Behandlung von Hautveränderungen, wie sie z. B. bei der Hautalterung auftreten (wie Ausbildung von Trockenheitsfältchen, Schlaffheit und Ausbildung von Falten und Fältchen). Weiterhin vorteilhaft eignen sich Collagen bzw. Wirkstoffe, die die Collagensynthese steigern, wie Ginkgo Biloba bzw. Ginkgo-Extrakte, Vitamin C, Creatin, um die Haut zu festigen und zu straffen.

Vorteilhaft ist ferner die Verwendung von erfindungsgemäßen Zubereitungen, welche Ascorbinsäure und/oder Derivate und/oder UV-Filtersubstanzen enthalten, zur kosmetischen Behandlung von Hautalterungserscheinungen (Ausbildung von Trockenheitsfältchen, Schlaffheit und Ausbildung von Falten und Fältchen).

Die Wasserphase der Zubereitungen A oder B gemäß der vorliegenden Erfindung kann vorteilhaft übliche kosmetische Hilfsstoffe enthalten, wie beispielsweise Alkohole, insbesondere solche niedriger C-Zahl, vorzugsweise Ethanol und/oder Isopropanol, Polymere, Schaumstabilisatoren, Elektrolyte sowie insbesondere ein oder mehrere Verdickungsmittel, welches oder welche vorteilhaft gewählt werden können aus der Gruppe Siliciumdioxid, Aluminiumsilikate, Magnesium Aluminium Silikate, Polysaccharide bzw. deren Derivate, z. B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole [von der Fa. Noveon], beispielsweise Carbopole der Typen 980, 981, 1382, 2984, 5984, ETD 2020, ETD 2050, Pemulen TR-1 oder -2, Ultrez 10, jeweils einzeln oder in Kombination.

Auch sog. Moisturizer sind vorteilhaft zu verwenden. Als Moisturizer werden Stoffe oder Stoffgemische bezeichnet, welche kosmetischen Zubereitungen die Eigenschaft verleihen, nach dem Auftragen bzw. Verteilen auf der Hautoberfläche die Feuchtigkeitsabgabe der Hornschicht (auch transepidermal water loss (TEWL) genannt) zu reduzieren und/oder die Hydratation der Hornschicht positiv zu beeinflussen.
Vorteilhafte Moisturizer im Sinne der vorliegenden Erfindung sind beispielsweise Glycerin, Milchsäure und/oder Lactate, insbesondere Natriumlactat, Butylenglykol, Propylenglykol, Biosaccaride Gum-1, Glycine Soja, Ethylhexyloxyglycerin, Pyrrolidoncarbonsäure und Harnstoff.

Die erfindungsgemäßen kosmetischen Zubereitungen können ferner vorteilhaft Füllstoffe enthalten, welche z. B. die sensorischen und kosmetischen Eigenschaften der Formulierungen weiter verbessern und beispielsweise ein samtiges oder seidiges Hautgefühl hervorrufen oder verstärken. Vorteilhafte Füllstoffe im Sinne der vorliegenden Erfindung sind Stärke und Stärkederivate (wie z. B. Tapiocastärke, Distärkephosphat, Aluminium- bzw. Natrium-Stärke Octenylsuccinat und dergleichen), Pigmente, die weder hauptsächlich UV-Filter- noch färbende Wirkung haben (wie z. B. Bornitrid etc.) und/oder Aerosile^{®} (CAS-Nr. 7631-86-9) sowie Lauroyl Lysine, Polymethylsilesquioxane, Polymethylmethacrylate, Polymethylmethacrylate Crosspolymer, Nylon, Talkum, Mica,

Erfindungsgemäß besonders bevorzugt sind Lauroyllysine und/oder Polymethylsilesquioxane, als Sonsorikadditive zu wählen.

Feine Fältchen sieht man, weil sich die Lichtreflexion auf der Hautoberfläche von der in den Faltensenken unterscheidet. Es ist daher vorteilhaft im Sinne der vorliegenden Erfindung, lichtstreuende Pigmente einzusetzen, die die Faltentiefe optisch reduzieren, indem sie den Anteil an diffus reflektiertem Licht in der Falte reduzieren, wodurch die Haut glatter und natürlicher aussieht. Auf diese Weise läßt sich auch übermäßiger Glanz der Haut optisch reduzieren. Vorteilhaft zu diesem Zweck zu verwenden ist speziell beschichtetes Silica - z. B. ganz besonders vorteilhaft Silica LDP (Silica + Titanium Dioxide + Iron Oxides), welches unter dem Handelsnamen Ronasphere LDP von Merck erhältlich ist.
Ist ist daher bevorzugt Silica LDP der dekorativen Zbereitung zu zusetzen.

Es ist erfindungsgemäß bevorzugt, den Gehalt an UV-Filtersubstanzen (eine oder mehrere Verbindungen) kleiner als 5 Gew.-%, insbesondere kleiner als 2 Gew.-% zu wählen, jeweils bezogen auf das Gesamtgewicht der Zubereitung. Ganz besonders vorteilhaft sind Zubereitungen im Sinne der vorliegenden Erfindung, welche frei von UV-Filtersubstanzen sind.

Für den Fall, dass ein Gehalt an UV-Filtersubstanzen gewünscht ist, werden diese vorteilhaft aus einer oder mehreren der folgenden Gruppen gewählt: UV-A-, UV-B- und/oder Breitbandfiltersubstanzen sowie organische und/oder anorganische Pigmente als UV-Filtersubstanzen.
Anorganische Pigmente wie Titandioxid besonders bevorzugt Titandioxid AQ-IP der Fa. Unichema.

Besonders vorteilhafte UV-Filtersubstanzen im Sinne der vorliegenden Erfindung sind:
- Dibenzoylmethanderivate, insbesondere das 4-(tert.-Butyl)-4'-methoxydibenzoylmethan (CAS-Nr. 70356-09-1), welches von Givaudan unter der Marke Parsol^{®} 1789 und von Merck unter der Handelsbezeichnung Eusolex® 9020 verkauft wird.
- Phenylen-1,4-bis-(2-benzimidazyl)-3,3'-5,5'-tetrasulfonsäure und ihre Salze, besonders die entsprechenden Natrium-, Kalium- oder Triethanolammonium-Salze, insbesondere das Phenylen-1,4-bis-(2-benzimidazyl)-3,3'-5,5'-tetrasulfonsäure-bis-natriumsalz mit der INCI-Bezeichnung Disodium Phenyl Dibenzimidazol Tetrasulfonat (CAS-Nr.: 180898-37-7), welches beispielsweise unter der Handelsbezeichnung Neo Heliopan AP bei Symrise erhältlich ist;
- Salze der 2-Phenylbenzimidazol-5-sulfonsäure, wie ihr Natrium-, Kalium- oder ihr Triethanolammonium-Salz sowie die Sulfonsäure selbst mit der INCI Bezeichnung Phenylbenzimidazole Sulfonsäure (CAS.-Nr. 27503-81-7), welches beispielsweise unter der Handelsbezeichnung Eusolex 232 bei Merck oder unter Neo Heliopan Hydro bei Symrise erhältlich ist;
- 1,4-di(2-oxo-10-Sulfo-3-bornylidenmethyl)-Benzol (auch: 3,3'-(1,4-Phenylendimethylene)-bis-(7,7-dimethyl-2-oxo-bicyclo-[2.2.1]hept-1-ylmethan Sulfonsäure) und dessen Salze (besonders die entprechenden 10-Sulfato-verbindungen, insbesondere das entsprechende Natrium-, Kalium- oder Triethanolammonium-Salz), das auch als Benzol-1,4-di(2-oxo-3-bornylidenmethyl-10-sulfonsäure) bezeichnet wird. Benzol-1,4-di(2-oxo-3-bornylidenmethyl-10-sulfonsäure) hat die INCI-Bezeichnung Terephtalidene Dicampher Sulfonsäure (CAS.-Nr.: 90457-82-2) und ist beispielsweise unter dem Handelsnamen Mexoryl SX von der Fa. Chimex erhältlich;
- Sulfonsäure-Derivate des 3-Benzylidencamphers, wie z. B. 4-(2-Oxo-3-bornylidenmethyl)-benzolsulfonsäure, 2-Methyl-5-(2-oxo-3-bornylidenmethyl)sulfonsäure und deren Salze.
- Benzoxazol-Derivate, wie z. B. das 2,4-bis-[5-1 (dimethylpropyl)benzoxazol-2-yl-(4-phenyl)-imino]-6-(2-ethylhexyl)-imino-1,3,5-triazin mit der CAS Nr. 288254-16-0, welches bei 3V Sigma unter der Handelsbezeichnung Uvasorb® K2A erhältlich ist.
- Hydroxybenzophenone, z. B. der 2-(4'-Diethylamino-2'-hydoxybenzoyl)-benzoesäurehexylester (auch: Aminobenzophenon), welcher unter der Handelsbezeichnung Uvinul A Plus bei der Fa. BASF erhältlich ist.
- Triazinderivate, wie z. B. 2,4-Bis-{[4-(2-Ethyl-hexyloxy)-2-hydroxy]-phenyl}-6-(4-methoxyphenyl)-1,3,5-triazin (INCI: Bis-Ethylhexyloxylphenol Methoxyphenyl Triazin), welches unter der Handelsbezeichnung Tinosorb® S bei der CIBA-Chemikalien GmbH erhältlich ist; Dioctylbutylamidotriazon (INCI: Diethylhexyl Butamido Triazone), welches unter der Handelsbezeichnung UVASORB HEB bei Sigma 3V erhältlich ist; 4,4',4"-(1,3,5-Triazin-2,4,6-triyltriimino)-tris-benzoësäure-tris(2-ethylhexylester), auch: 2,4,6-Tris-[anilino-(p-carbo-2'-ethyl-1'-hexyloxy)]-1,3,5-triazin (INCI: Ethylhexyl Triazone), welches von der BASF Aktiengesellschaft unter der Warenbezeichnung UVINUL® T 150 vertrieben wird; 2-[4,6-Bis(2,4-dimethylphenyl)-1,3,5-triazin-2-yl]-5-(octyloxy)phenol (CAS Nr.: 2725-22-6).
- Benzotriazole, wie z. B. 2,2'-Methylen-bis-(6-(2H-benzotriazol-2-yl)-4-(1,1,3,3-tetramethylbutyl)-phenol) (INCI: Methylene Bis-Benztriazolyl Tetramethylbutylphenol), welches z. B. unter der Handelsbezeichnung Tinosorb® M bei der CIBA-Chemikalien GmbH erhältlich ist.
- 3-Benzylidencampher-Derivate, vorzugsweise 3-(4-Methylbenzyliden)campher, 3-Benzylidencampher;
- 4-Aminobenzoesäure-Derivate, vorzugsweise 4-(Dimethylamino)-benzoesäure(2-ethylhexyl)ester, 4-(Dimethylamino)benzoesäureamylester;
- Ester der Benzalmalonsäure, vorzugsweise 4-Methoxybenzalmalonsäuredi(2-ethylhexyl)-ester;
- Ester der Zimtsäure, vorzugsweise 4-Methoxyzimtsäure(2-ethylhexyl)ester, 4-Methoxyzimtsäureisopentylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon sowie
- an Polymere gebundene UV-Filter
- Ethylhexyl-2-cyano-3,3-diphenylacrylat (Octocrylen), welches von BASF unter der Bezeichnung Uvinul^{®} N 539 T erhältlich ist.
- Ethylhexylmethoxycinnamate (Uvinol MC 80 von der Firma BASF)

Es ist ferner vorteilhaft im Sinne der vorliegenden Erfindung, wenn mindestens eine der Zubereitungen einen oder mehrere Filmbildner bzw. Polymere enthält, insbesondere um einen nachvollziehbaren Straffungseffekt auf der Haut zu erzielen, der durch deren Filmbildungseigenschaften hervorgerufen werden kann.
Hierzu eignen sich z. B.
Polyurethane (z. B. die Avalure® -Typen von Goodrich), Dimethicone Copolyol Polyacrylate (Silsoft Surface® von der Witco Organo Silicones Group), PVP/VA (VA = Vinylacetat) Copolymer (Luviscol VA 64 Powder der BASF), C₂₀₋₄₀ Carbonsäure mit Polyethylen (Performacid 350 von der Fa. New Phase Technologies) sowie Filmbildner aus der Gruppe der Polymere auf Basis von Polyvinylpyrrolidon (PVP):

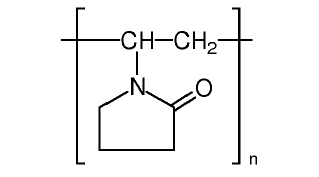

Besonders bevorzugt werden Copolymere des Polyvinylpyrrolidons eingesetzt, beispielsweise das PVP Hexadecen Copolymer und das PVP Eicosen Copolymer, welche unter den Handelsbezeichnungen Antaron V216 und Antaron V220 bei der GAF Chemicals Cooperation erhältlich sind, sowie das Tricontayl PVP und dergleichen mehr.

Insbesondere bevorzugt sind wasserlösliche Polymere wie Vinylpyrrolidon/Vinylacetat- (VP/VA-) Copolymere und Natriumpolystyrensulfonat.

Es ist ferner vorteilhaft im Sinne der vorliegenden Erfindung, wenn mindestens eine der Zubereitungen - insbesondere bevorzugt die Foundation - ein oder mehrere anorganische und/oder organische Pigmente und/oder einen oder mehrere Farbstoffe enthält.
Im Bereich der kosmetischen Mittel sind zur Färbung des Produktes oder zur Färbung des zu behandelnden "Objektes" (Haut, Haare, Lippen) sowohl lösliche Farbmittel (im Rahmen der vorliegenden Beschreibung auch als Farbstoffe bezeichnet) und unlösliche Farbmittel (im Rahmen der vorliegenden Beschreibung auch als Pigmente bezeichnet) zugelassen.

Die Farbstoffe können sowohl synthetischen als auch natürlichen Ursprungs sein.

Bevorzugt im Sinne der vorliegenden Erfindung sind Pigmentmischungen aus Weiß-Pigmenten (z. B. Kaolin, Titandioxid oder Zinkoxid) und anorganischen Pigmenten (z. B. EisenoxidPigmente, Chromoxide), wobei die Pigmente beschichtet ("gecoatet") oder unbeschichtet vorliegen können.

Bei den Farbpigmenten kommen vorzugsweise die Eisenoxide zur Verwendung

Es kommen außerdem vorzugsweise Weißpigmente zur Anwendung, dies sind Pigmente, deren optische Wirkung vorwiegend auf nicht selektiver Lichtstreuung beruht (DIN 55944: 2003-11). Von den chemisch oft sehr ähnlichen Füllstoffen unterscheiden sich anorganische Weißpigmente vor allem durch ihre im allgemeinen höhere Brechzahl und, damit verbunden, ihr höheres Streuvermögen sowie nach DIN 55943: 2001-10 ihre Anwendung*.*

Ideale Weißpigmente zeigen keine Absorption im Bereich des sichtbaren Lichts, dafür hohes Streuvermögen, das hohes Deckvermögen zur Folge hat. Das Streuvermögen ist umso größer, je größer die Differenz zwischen der Brechzahl des Weißpigmentes und der des umgebenden Mediums ist.

Tabelle: Brechzahlen und Colour Index verschiedener Weißpigmente.

| Name | Formel | Brechzahl n | Colour Index |
|---|---|---|---|
| *Weißpigmente* | | | |
| Titandioxid-Pigmente | TiO₂ | | PW 6, 77891 |
| - Anatas-Pigmente | | 2,55 | |
| - Rutil-Pigmente | | 2,75 | |
| Zinkoxid (Zinkweiß) | ZnO | 1,95-2,1 | PW 4,77947 |
| *Füllstoffe* | | | |

Bevorzugt in der kosmetischen Zubereitung zum Einsatz kommen Titandioxide als Weißpigment.

Zusätzlich vorteilhaft können die kosmetischen Zubereitungen Effektpigmente enthalten, die neben Farbe eine zusätzliche Eigenschaft, wie z.B. Winkelabhängigkeit der Farbe (changieren, Flop), Glanz (nicht Oberflächenglanz) oder Textur, verleihen.
Die bedeutendste Gruppe der Effektpigmente stellen die Glanzpigmente dar, zu denen nach DIN 55944: 2003-11 die Metalleffektpigmente und die Perlglanzpigmente gehören (siehe nachstehende Tabelle). Einige spezielle Effektpigmente lassen sich diesen beiden Gruppen nicht zuordnen, z.B. plättchenförmiges Graphit, plättchenförmiges Eisenoxid und mikronisiertes Titandioxid. Letzteres erzeugt nicht einen Glanzeffekt, sondern einen winkelabhängigen Lichtstreueffekt.

Bei den Glanzpigmenten nach DIN 55943: 2001-10 handelt es sich vorwiegend plättchenförmige Effektpigmente. Parallel orientiert zeigen Glanzpigmente einen charakteristischen Glanz. Die optische Wirkung von Glanzpigmenten beruht auf der gerichteten Reflexion an metallischen Teilchen (Metalleffektpigmente), an transparenten Teilchen mit hoher Brechzahl (Perlglanzpigmente) oder auf dem Phänomen der Interferenz (Interferenzpigmente) (DIN 55944: 2003-11).

Beispiele für bevorzugte handelübliche Effektpigmente sind : Timiron® von Merck , Iriodin® von Merck (Perl- und Farbglanzpigmente für dekorative technische Anwendungen), Xirallic® von Merck (Farbintensive Kristalleffektpigmente)

Weiterhin können optional organische Farbstoffe zum Einsatz kommen. Dies sind im Anwendungsmedium praktisch unlösliche organische Farbmittel. Nach DIN 55944: 1990-04 können organische Pigmente nach chemischen Gesichtspunkten in Azopigmente und polycyclische Pigmente und nach koloristischen Gesichtspunkten in Bunt- oder Schwarzpigmente eingeteilt werden.

Polycyclische Pigemente umfassen nach Nach DIN 55944 p. P. alle organischen Nicht-Azopigmente, die durch aromatische und/oder heteroaromatische Ringsysteme charakterisiert sind.

Einzelne oder mehrere der zuvor benannten bevorzugten Bestandteile sind je nach Anwendungszweck, d.h. zur Hautpflege oder zu dekorativen Zwecken, entsprechend der erfindungsgemäßen Kombination dann in mindestens einer der Zubereitungen enthalten, bevorzugt nur in einer der getrennt gelagerten Zubereitungen vorhanden.

Die nachfolgenden Beispiele sollen die vorliegende Erfindung verdeutlichen, ohne sie einzuschränken. Die Zahlenwerte in den Beispielen bedeuten Gewichtsprozente, bezogen auf das Gesamtgewicht der jeweiligen Zubereitungen, sofern nichts anderes bezeichnet ist.

Der entscheidende Unterschied zwischen den mindestens zwei Zubereitungen ist, dass es sich bei der hautpflegenden Formulierung A um kein dekoratives Produkt handelt, d.h. im Gegensatz zur Zubereitung B, dem Make-up, enthält es keine Farbstoffe, was vorteilhaft auch an der Färbung der beiden Zubereitungen zu sehen ist. Die hautpflegende Zubereitung A ist vorteihaft weiß oder transparent und die dekorative Zubereitung B ist vorteilhaft nicht weiß sondern bräunlich, beige gefärbt, wobei die Färbung in unterschiedlichen Nuancen und Ausprägung erfolgen kann.

### Beispiele

### 1. Zubereitung zur Pflege der Haut (Zubereitung A)

### O/W Emulsionen

| | 1 | 2 | 3 | 4 | 5 | 6 | 7 |
|---|---|---|---|---|---|---|---|
| Glycerinmonostearat SE | | | 3,00 | | | 1,50 | |
| Glycerylstearatcitrat | 2,00 | | | | | | |
| Stearinsäure | | | 0,75 | | | | |
| PEG-40 Stearat | | | | | | 2,00 | |
| PEG-100 Stearat | | | 1,50 | | | | |
| Cetyl Phosphat | | | 0,75 | | | | |
| Stearyl Alkohol | | | 3,00 | | | 2,00 | |
| Cetyl Alkohol | 2,50 | | | | | | |
| Sucrose Palmitate¹ | | 5,0 | | 2,0 | 1,0 | | 3,0 |
| Bis-Ethylhexyloxyphenol Methoxyphenyl Triazin | | 1,00 | | | | 1,00 | 0,50 |
| Disodium Phenyl Dibenzimidazol Tetrasulfonat | | | | 1,00 | | 2,00 | |
| Ethylhexyl Triazon | 2,00 | | | 2,00 | | 2,00 | |
| Diethylhexyl Butamido Triazon | | 2,00 | | | | | |
| Ethylhexyl Methoxycinnamat | | 3,50 | | 10,00 | | | |
| Octocrylen | | | | 5,00 | 2,00 | 7,50 | 2,50 |
| Titandioxid T 805 | | 1,50 | | | 1,00 | 0,50 | |
| Titandioxid MT-100Z | 1,00 | | | 3,00 | 1,00 | | |
| C₁₂₋₁₅ Alkyl Benzoat | | 2,00 | | | | 7,00 | 5,00 |
| Dicaprylylether | | | 3,50 | | 1,00 | | |
| Butylenglycol Dicaprylat/ Dicaprat | 2,00 | | | 5,00 | 1,00 | | |
| Cetearyl Isononanoate | | 2,00 | | | | 2,00 | 2,00 |
| Dimethicon | 5,00 | 4,50 | 1,00 | 3,00 | 2,00 | | |
| Cyclomethicon | 5,00 | 15,0 | | 10,50 | 25,00 | | 0,50 |
| Dimethicon / Vinyl Dimethicon Crosspolymer | 4,00 | | | | | | 0,50 |
| Shea Butter | 1,50 | 0,5 | | 1,00 | 1,00 | | 1,00 |
| Methyl Methaacrylate Crosspolymer | 1,00 | | | | | | 1,00 |
| Nylon -12 | 2,00 | | | | | 2,00 | |
| Polymethylsilesquioxane | | 2,00 | | 2,00 | 1,00 | 1,00 | |
| Lauroyl Lysine | | 3,00 | | 2,00 | 2,00 | | 1,00 |
| Titandioxid (CI 77891) | | 3,00 | | | 4,50 | 6,00 | |
| Beschichtetes Silica ² | 1,00 | | | 1,00 | | | 1,0 |
| Effektpigmente (z.B. beschichtetes Mica)³ | | 1,00 | 0,50 | | 0,04 | | 0,27 |
| Glycerin | 3,00 | 7,50 | | 7,50 | 5,00 | | 10,00 |
| Butylenglycol | 7,00 | 5,00 | | | | 7,00 | |
| Vitamin A Palmitat | 0,50 | 0,1 | 0,25 | | | | 1,00 |
| Vitamin C Phosphat | 0,1 | 0,05 | 0,5 | 0,1 | 0,25 | 0,5 | 0,1 |
| Hyaluronsäure | 0,01 | 0,05 | 0,50 | 0,03 | 0,35 | 1,0 | 0,1 |
| Gingko Biloba | 0,1 | 0,2 | 1,50 | 0,25 | 0,10 | 2,00 | 1,00 |
| Natriumpolystyrensulfonate | 0,2 | 0,5 | 0,25 | 0,1 | 0,2 | 2,0 | 1,0 |
| VP/VA Copolymer | 0,5 | 0,2 | 0,25 | 1,0 | 0,2 | 1,0 | 2,0 |
| PVP Hexadecen Copolymer | 0,5 | | | | 0,10 | | 1,00 |
| Xanthan Gummi | 0,5 | 0,3 | 0,3 | | 0,2 | 0,5 | |
| Magnesium Aluminium Silikate | | 3,00 | | 2,00 | 2,00 | | 3,00 |
| DMDM Hydantoin | | 0,60 | 0,40 | 0,20 | | | |
| Parabene | 0,5 | | 0,25 | | 0,70 | | 0,70 |
| Phenoxyethanol | 1,00 | 0,40 | | 0,40 | 0,90 | | 0,60 |
| EDTA oder Tetra Sodium Iminodisuccinate | | 0,20 | 0,35 | 0,50 | 0,02 | | 0,03 |
| Ethanol | 2,00 | 3,00 | 3,00 | 1,50 | 3,00 | 5,00 | 1,00 |
| Parfüm | 0,20 | 0,20 | | | | 0,30 | 0,40 |
| Wasser | ad 100 | ad 100 | ad 100 | ad 100 | ad 100 | ad 100 | ad 100 |

| | | | | | | | |
|---|---|---|---|---|---|---|---|
| ¹ Arlatone V-175 von Uniqema (Phenoxyethanol + Methylparaben+ Ethylparaben + Butylparaben + Isobutylparaben+ Propylparaben + Sucrose Palmitate + Glyceryl Stearate + Sucrose + Xanthan Gum + Mannan + Glyceryl Stearate Citrate) ^{2 z.B.} Ronasphere LDP von Merck (Silica + Titanium Dioxide + Iron Oxides) ^{3 z.B.} Timiron von Merck (Mica & CI 77891) | | | | | | | |

### 2. Foundation (Zubereitung B)

### O/W Emulsionen

| | 1 | 2 | 3 | 4 | 5 | 6 | 7 |
|---|---|---|---|---|---|---|---|
| Glycerinmonostearat SE | | | 3,00 | | | 1,50 | |
| Glyceryl Stearat Citrat | 2,00 | | | | | | |
| Stearinsäure | | | 0,75 | | | | |
| PEG-40 Stearat | | | | | | 2,00 | |
| PEG-100 Stearat | | | 1,50 | | | | |
| Cetyl Phosphat | | | 0,75 | | | | |
| Stearyl Alkohol | | | 3,00 | | | 2,00 | |
| Cetyl Alkohol | 2,50 | | | | | | |
| Sucrose Palmitate ¹ | | 5,0 | | 2,0 | 1,0 | | 3,0 |
| Bis-Ethylhexyloxyphenol Methoxyphenyl Triazin | | 1,00 | | | | 1,00 | 0,50 |
| Disodium Phenyl Dibenzimidazol Tetrasulfonat | | | | 1,00 | | 2,00 | |
| Ethylhexyl Triazon | 2,00 | | | 2,00 | | 2,00 | |
| Diethylhexyl Butamido Triazon | | 2,00 | | | | | |
| Ethylhexyl Methoxycinnamat | | 3,50 | | 10,00 | | | |
| Octocrylen | | | | 5,00 | 2,00 | 7,50 | 2,50 |
| Titandioxid T 805 | | 1,50 | | | 1,00 | 0,50 | |
| Titandioxid MT-100Z | 1,00 | | | 3,00 | 1,00 | | |
| C₁₂₋₁₅ Alkyl Benzoat | | 2,00 | | | | 7,00 | 5,00 |
| Dicaprylylether | | | 3,50 | | 1,00 | | |
| Butylenglycol Dicaprylat/ Dicaprat | 2,00 | | | 5,00 | 1,00 | | |
| Cetearyl Isononanoate | | 2,00 | | | | 2,00 | 2,00 |
| Dimethicon | 5,00 | 4,50 | 1,00 | 3,00 | 2,00 | | |
| Cyclomethicon | 5,00 | 15,0 | | 10,50 | 25,00 | | 0,50 |
| Dimethicon / Vinyl Dimethicon Crosspolymer | 4,00 | | | | | | 0,50 |
| Shea Butter | 1,50 | 0,5 | | 1,00 | 1,00 | | 1,00 |
| Methyl Methaacrylate Crosspolymer | 1,00 | | | | | | 1,00 |
| Nylon -12 | 2,00 | | | | | 2,00 | |
| Polymethylsilesquioxane | | 2,00 | | 2,00 | 1,00 | 1,00 | |
| Lauroyl Lysine | | 3,00 | | 2,00 | 2,00 | | 1,00 |
| Titandioxid (CI 77891) | 9,00 | 6,00 | 7,00 | 6,00 | 4,50 | 3,00 | 10,00 |
| Pigmente (C177492, 77491,77499,77007) | 2,0 | 3,0 | 2,6 | 4,0 | 1,5 | 2,5 | 3,0 |
| Beschichtetes Silica² | | 0,50 | | 1,00 | 0,50 | | 1,0 |
| Effektpigmente (z.B. beschichtetes Mica) ³ | | 1,00 | 0,50 | | | | 0,27 |
| Glycerin | 3,00 | 7,50 | | 7,50 | 5,00 | | 10,00 |
| Butylenglycol | 7,00 | 5,00 | | | | 7,00 | |
| Vitamin A Palmitat | 0,50 | 0,1 | 0,25 | | | | 1,00 |
| Vitamin C Phosphat | 0,1 | 0,05 | 0,5 | 0,1 | 0,25 | 0,5 | 0,1 |
| Hyaluronsäure | 0,01 | 0,05 | 0,50 | 0,03 | 0,35 | 1,0 | 0,1 |
| Gingko Biloba | 0,1 | 0,2 | 1,50 | 0,25 | 0,10 | 2,00 | 1,00 |
| Natrium Polystyrene Sulfonate | 0,2 | 0,5 | 0,25 | 0,1 | 0,2 | 2,0 | 1,0 |
| VP/VA Copolymer | 0,5 | 0,2 | 0,25 | 1,0 | 0,2 | 1,0 | 2,0 |
| PVP Hexadecen Copolymer | 0,5 | | | | 0,10 | | 1,00 |
| Xanthan Gummi | 0,5 | 0,3 | 0,3 | | 0,2 | 0,5 | |
| Magnesium Aluminium Silikate | | 3,00 | | 2,00 | 2,00 | | 3,00 |
| DMDM Hydantoin | | 0,60 | 0,40 | 0,20 | | | |
| Parabene | 0,5 | | 0,25 | | 0,70 | | 0,70 |
| Phenoxyethanol | 1,00 | 0,40 | | 0,40 | 0,90 | | 0,60 |
| EDTA oder Tetra Sodium Iminodisuccinate | | 0,20 | 0,35 | 0,50 | 0,02 | | 0,03 |
| Ethanol | 3,00 | 3,00 | 2,00 | 1,00 | 3,00 | 5,00 | 2,50 |
| Parfüm | 0,20 | 0,20 | | | | 0,30 | 0,40 |
| Wasser | ad 100 | ad 100 | ad 100 | ad 100 | ad 100 | ad 100 | ad 100 |

| | | | | | | | |
|---|---|---|---|---|---|---|---|
| Arlatone V-175 von Uniqema (Phenoxyethanol + Methylparaben+ Ethylparaben + Butylparaben + Isobutylparaben+ Propylparaben + Sucrose Palmitate + Glyceryl Stearate + Sucrose + Xanthan Gum + Mannan + Glyceryl Stearate Citrate) ^{2 z.B.} Ronasphere LDP von Merck (Silica + Titanium Dioxide + Iron Oxides) ^{3 z.B..} Timiron von Merck (Mica & Cl 77891) | | | | | | | |

### 3. Foundation (Zubereitung B)

### W/O Emulsionen

| | 1 | 2 | 3 | 4 | 5 | 6 | 7 |
|---|---|---|---|---|---|---|---|
| Cetyl PEG/PPG -10/1 Dimethicon | | 3,00 | | 4,00 | 3,00 | 4,00 | 4,00 |
| Polyglyceryl-2-dipolyhydroxystearat | 5,00 | | | | | | |
| PEG-30-dipolyhydroxystearat | | | 5,00 | | | | |
| Bis-Ethylhexyloxyphenol Methoxyphenyl Triazin | | 1,00 | | | | 1,00 | 0,50 |
| Disodium Phenyl Dibenzimidazol Tetrasulfonat | | | | 1,00 | | 2,00 | |
| Ethylhexyl Triazon | 2,00 | | | 2,00 | | 2,00 | |
| Diethylhexyl Butamido Triazon | | 2,00 | | | | | |
| Ethylhexyl Methoxycinnamat | | 3,50 | | 10,00 | | | |
| Octocrylen | | | | 5,00 | 2,00 | 7,50 | 2,50 |
| Titandioxid T 805 | | 1,50 | | | 1,00 | 0,50 | |
| Titandioxid MT-100Z | 1,00 | | | 3,00 | 1,00 | | |
| Dicaprylylether | | | 3,50 | | 1,00 | | |
| Dicaprylyl Carbonate | | | | | | 4,00 | 4,00 |
| Squalane | | 5,00 | | | | | |
| Dimethicon | 5,00 | 4,50 | 1,00 | 3,00 | 2,00 | 4 | 5 |
| Cyclomethicon | 5,00 | 15,0 | | 10,50 | 25,00 | 12 | 10 |
| Dimethicon / Vinyl Dimethicon Crosspolymer | 4,00 | | | | | | 1,00 |
| Shea Butter | 1,50 | 0,5 | | 1,00 | 1,00 | | 2,00 |
| Methyl Methaacrylate Crosspolymer | 1,00 | | | | | | 1,00 |
| Nylon -12 | 2,00 | | | | | 2,00 | |
| Polymethylsilesquioxane | | 2,00 | | 2,00 | 1,00 | 1,00 | |
| Lauroyl Lysine | | 3,00 | | 2,00 | 2,00 | | 1,00 |
| Titandioxid (Cl 77891) | 9,00 | 6,00 | 7,00 | 6,00 | 4,50 | 3,00 | 10,00 |
| Pigmente (Cl77492, 77491,77499,77007) | 2,0 | 3,0 | 2,6 | 4,0 | 1,5 | 2,5 | 3,0 |
| Beschichtetes Silica² | | 0,50 | | 1,00 | 0,50 | | 1,0 |
| Effektpigmente (z.B. beschichtetes Mica)³ | | 1,00 | 0,50 | | | | 0,27 |
| Glycerin | 3,00 | 7,50 | | 7,50 | 5,00 | | 10,00 |
| Butylenglycol | 7,00 | 5,00 | | | | 7,00 | |
| Vitamin A Palmitat | 0,50 | 0,1 | 0,25 | | | | 1,00 |
| Vitamin C Phosphat | 0,1 | 0,05 | 0,5 | 0,1 | 0,25 | 0,5 | 0,1 |
| Hyaluronsäure | 0,01 | 0,05 | 0,50 | 0,03 | 0,35 | 1,0 | 0,1 |
| Gingko Biloba | 0,1 | 0,2 | 1,50 | 0,25 | 0,10 | 2,00 | 1,00 |
| Natrium Polystyrene Sulfonate | 0,2 | 0,5 | 0,25 | 0,1 | 0,2 | 2,0 | 1,0 |
| VP/VA Copolymer | 0,5 | 0,2 | 0,25 | 1,0 | 0,2 | 1,0 | 2,0 |
| PVP Hexadecen Copolymer | 0,5 | | | | 0,10 | | 1,00 |
| Magnesium Sulfate | | | | 1,00 | 1,00 | | |
| Natrium Chlorid | 2,00 | 1,00 | 2,00 | | | 2,00 | 2,00 |
| Propylene Carbonate | 0,12 | 0,12 | 0,12 | 0,12 | 0,12 | 0,12 | 0,12 |
| Disteardimonium Hectorite | 0,5 | 0,5 | 0,5 | 0,5 | 0,5 | 0,5 | 0,5 |
| DMDM Hydantoin | | 0,60 | 0,40 | 0,20 | | | |
| Parabene | 0,5 | | 0,25 | | 0,70 | | 0,70 |
| Phenoxyethanol | 1,00 | 0,40 | | 0,40 | 0,90 | | 0,60 |
| EDTA oder Tetra Sodium Iminodisuccinate | | 0,20 | 0,35 | 0,50 | 0,02 | | 0,03 |
| Ethanol | 3,00 | 3,00 | 2,00 | 1,00 | 3,00 | 5,00 | 2,50 |
| Parfüm | 0,20 | 0,20 | | | | 0,30 | 0,40 |
| Wasser | ad 100 | ad 100 | ad 100 | ad 100 | ad 100 | ad 100 | ad 100 |

| | | | | | | | |
|---|---|---|---|---|---|---|---|
| ^{2 z.B.} Ronasphere LDP von Merck (Silica + Titanium Dioxide + Iron Oxides) ^{3 z.B..} Timiron von Merck (Mica & Cl 77891) | | | | | | | |

Die unter 1. aufgeführten hautpflegenden Zubereitungen lassen sich perfekt mit den unter 2. bzw. 3. aufgeführten dekorativen Zubereitungen in einem erfindungsgemäßen Dispenser bereit stellen.

## Patentansprüche

1. Kosmetisches Kombinationsset umfassend
a.) einen Mehrkammerapplikator zur zeitgleichen und getrennten Ausbringung mindestens zwei im Applikator getrennt enthaltener Zubereitungen, umfassend eine Verschlussvorrichtung, die die Zubereitungen hermetisch voneinander und der Umgebung trennt und die getrennte Ausgabeöffnungen für jede Kammer umfasst,
b.) wobei mindestens eine der Zubereitungen eine hautpflegende Zubereitung und
c.) mindestens eine andere Zubereitung eine dekorative Zubereitung und
d.) die hautpflegenden und die dekorativen Zubereitungen sich in mindestens einem Inhaltstoff voneinander unterscheiden und
e.) die hautpflegenden und die dekorativen Zubereitungen so gewählt werden, dass sie sich bei beim Verteilen, insbesondere auf der Haut, homogen mischen.

2. Kombinationsset nach Anspruch 1, **dadurch gekennzeichnet, dass** die hautpflegende Zubereitung eine O/W-Emulsion und die dekorative Zubereitung eine W/S- oder O/W-Emulsion ist.

3. Kombinationsset nach Anspruch 1, **dadurch gekennzeichnet, dass** die dekorative Zubereitung eine W/O-Emulsion ist.

4. Kombinationsset nach einem der vorstehenden Ansprüche, **dadurch gekennzeichnet, dass** der Applikator aus einem Behältnis mit mindestens zwei getrennten Zubereitungslagern besteht.

5. Kombinationsset nach einem der vorstehenden Ansprüche, **dadurch gekennzeichnet, dass** die hautpflegende Zubereitung jeweils einen oder mehrere Bestandteile gewählt aus der Gruppe der hautpflegenden Wirkstoffe, Polymere mit filmbildenden Eigenschaften und Emulgatoren sowie gegebenenfalls Sensorikadditive und UV-Filter umfasst.

6. Kombinationsset nach Anspruch 5, **dadurch gekennzeichnet, dass** die hautpflegende Zubereitung
a.) als hautpflegende Wirkstoffe Ascorbinsäure und/oder deren Derivate, Hyaluronsäure, Gingko Biloba, Kollagen und/oder Wirkstoffe, die die Kollagensynthese stimulieren, gewählt werden,
b.) als Polymere mit filmbildenden Eigenschaften VP/VA Copolymere und/oder Natriumpolystyrensulfonate gewählt werden,
c.) als Emulgator Sucrose Ester, Sucrosepalmitate, Glycerylstearate und/oder Glycerylstearatcitrate gewählt werden und/oder
d.) als Sensorikadditive Lauroyllysine, Polymethylsilesquioxane, Polymethylmethacrylate Crosspolymer, Nylon, Talkum, Mica und/oder Stärkederivate gewählt werden.

7. Kombinationsset nach Anspruch 5 oder 6, **dadurch gekennzeichnet, dass** die hautpflegende Zubereitung
a.) als hautpflegende Wirkstoffe Ascorbinsäure, bevorzugt Vitamin C Phosphat, Hyaluronsäure und Gingko Biloba und/oder Mischungen daraus gewählt werden,
b.) als Polymere mit filmbildenden Eigenschaften VP/VA Copolymere und/oder Natriumpolystyrensulfonate gewählt werden,
c.) als Emulgator Sucrosepalmitat in Abmischung mit Glycerystearat und Glycerylstearatcitrate gewählt werden und/oder
d.) als Sensorikadditive Lauroyllysin und/oder Polymethylsilesquioxan gewählt werden.

8. Kombinationsset nach Anspruch 5, 6 oder 7, **dadurch gekennzeichnet, dass** die
a.) hautpflegenden Wirkstoffe zu Anteilen von 0,01 - 10 Gew.%, bevorzugt von 0,01 - 2,5 Gew.%, bezogen auf die Gesamtmasse der hautpflegenden Zubereitung, zugesetzt sind,
b.) Polymere mit filmbildenden Eigenschaften zu 0,1 - 10 Gew.%, insbesondere zu 0,1 - 2,5 Gew.%, bezogen auf die Gesamtmasse der hautpflegenden Zubereitung, zugesetzt sind,
c.) Emulgatoren zu 0,01 - 3 Gew.%, insbesondere zu 0,01 - 1,5 Gew.%, bezogen auf die Gesamtmasse der hautpflegenden Zubereitung, zugesetzt sind.

9. Kombinationsset nach einem der vorstehenden Ansprüche, **dadurch gekennzeichnet, dass** die dekorative Zubereitung jeweils mindestens einen oder mehrere Bestandteile gewählt aus der Gruppe der Farbstoffe oder Pigmente und Emulgatoren sowie gegebenfalls Füllstoffe, Sensorikadditive und/oder Filmbildner umfasst.

10. Kombinationsset nach einem der vorstehenden Ansprüche, **dadurch gekennzeichnet, dass** in der dekorative Zubereitung
a.) als Farbstoff bzw. Pigment pigmentäre Farbstoffe auf Basis Eisenoxide und/oder Titandioxide, gecoatet oder nicht gecoatet, Effektpigmente, Perlglanzpigmente und/oder Metalleffektpigmente, insbesondere gecoatetes Mica, gewählt werden,
b.) als Emulgatoren eine Mischung aus Cetyl PEG/PPG-10/1 Dimethicone bei W/S-Emulsionen oder Sucrose Ester, bevorzugt Sucrose Palmitate in Abmischung mit Glycerylstearate und Glycerylstearatcitrate, bei O/W-Emulsionen gewählt werden,
c.) als Füllstoff gecoatetes Silica gewählt wird und/oder
d.) als Sensorikadditive Lauroyllysine, Polymethylsilesquioxane, Polymethylmethacrylate, Nylon, Talkum, Mica und/oder Stärkederivate gewählt werden.

11. Kombinationsset nach Anspruch 9 oder 10, **dadurch gekennzeichnet, dass** die
a.) Farbstoff- bzw. Pigmentanteile zu einem Anteil von 2 Gew.% und mehr, besonders bevorzugt 10 Gew.% und mehr, bezogen auf die Gesamtmasse der dekorativen Zubereitung, zugesetzt sind und
b.) Emulgatoren zu 0,01 - 3 Gew.%, insbesondere zu 0,01 - 1,5 Gew.%, bezogen auf die Gesamtmasse der dekorativen Zubereitung, zugesetzt sind.

12. Kombinationsset nach einem der vorstehenden Ansprüche umfassend keine Reinigungsmittel und/oder Tenside.

13. Verwendung des Kombinationssets nach einem der vorstehenden Ansprüche zur zeitgleichen Applikation mindestens einer hautpflegenden und mindestens einer dekorativen kosmetischen Zubereitung.

14. Verwendung des Kombinationsset nach einem der vorstehenden Ansprüche zur zeitgleichen Pflege und dekorativen Behandlung der Haut.

15. Verwendung des Kombinationsset nach einem der vorstehenden Ansprüche zur kosmetischen Straffung der Haut.

16. Verwendung des Kombinationsset nach einem der vorstehenden Ansprüche zur kosmetischen Hautglättung.
